# EUROPÄISCHE PATENTANMELDUNG

(11) **EP 0 648 799 A1**
(43) Veröffentlichungstag der Anmeldung: **19.04.1995**
(21) Anmeldenummer: 94110931.6
(22) Anmeldetag: 14.07.1994
(51) Int. Cl.: C08H 5/00, C07G 17/00, A61K 35/10

(54) **Verfahren zur Herstellung von Edelmetallhuminaten**

(30) Priorität: 16.10.1993 DE 4335370
(71) Anmelder: RÜTGERSWERKE AKTIENGESELLSCHAFT, 60326 Frankfurt (DE)
(72) Erfinder: Seubert, Bernhard, D-68535 Edingen-Neckarhausen (DE); Haase, Anton Franz, Dr., D-64367 Mühltal (DE)

(57) **Zusammenfassung**

Im erfindungsgemäßen Verfahren zur Herstellung von Edelmetallhuminaten wird eine mehrwertige phenolische Verbindung in alkalischer wäßriger Lösung elektrochemisch oder plasmachemisch oxidiert, wobei in der Reaktionskammer nach dem Start der Oxidationsreaktion der alkalischen Lösung der mehrwertigen phenolischen Verbindung eine Lösung oder ein Hydrosol eines entsprechenden Edelmetallsalzes zugegeben werden. Nach Beendigung der Oxidationsreaktion wird das Reaktionsgemisch auf einen pH-Wert im Bereich von 4 bis 6 eingestellt.

## Beschreibung

Die Erfindung betrifft ein Verfahren zur Herstellung stabiler Verbindungen zwischen niedermolekularen Huminstoffen und Metallen, vorwiegend Edelmetallen.

Edelmetallverbindungen und deren Zubereitungen finden in verschiedenen Gebieten der Medizin Anwendung.

Silber dient als Breitbandbiozid, als Wundbehandlungsmittel für Verbrennungen und als Schorfbildner.

Gold wird in der Therapie von Erkrankungen des rheumatischen Formenkreises eingesetzt.

Platin findet in der Anwendungsform cis-Platin Einsatz in der Behandlung maligner Tumoren.

Den gesicherten Wirkungen von Edelmetallverbindungen stehen bedingt durch hohe Toxizität starke Nebenwirkungen entgegen, welche die therapeutische Breite einengen.

Andererseits ist aus DE-A 41 29 872 bekannt, daß Huminsäure-Metall-Verbindungen physiologisch gut verträglich sind und auch im medizinischen Bereich eingesetzt werden.

Es war zu erwarten, daß auch die medizinische Anwendbarkeit von Edelmetallverbindungen verbessert wird, wenn die Edelmetalle in Form von Verbindungen mit Huminsäure, insbesondere mit Huminsäure mit niedrigem Molekulargewicht vorliegen und eingesetzt werden.

Leider zeigte sich, daß durch übliche Herstellungsverfahren wie z. B. durch Zugabe von Metallsalzlösungen zu wäßrigen Huminsäurelösungen oder partiell neutralisierten Alkalihuminatlösungen mit Edelmetallsalzen keine stabilen Huminate hergestellt werden können. Die entsprechenden Produkte oder Komplexe zersetzen sich nach kurzer Zeit und scheiden die Edelmetalle aus. Dies ist in Übereinstimmung mit Beobachtungen anderer Fachleute.

So ist z. B. aus Chem. Geol 102 (1 - 4), 53 - 71 (C. A. 118(12): 106459b) bekannt, daß Gold(III)-chlorid durch Huminstoffe unter allen beobachteten Bedingungen innerhalb von 8 bis 14 d zu elementarem, kolloidalem Gold reduziert wird.

Es ist daher Aufgabe der vorliegenden Erfindung, Edelmetallhuminate bereitzustellen, die so stabil sind, daß sie auch in pharmazeutischen Präparaten eingesetzt werden können.

Die Lösung der Aufgabe erfolgt durch ein Verfahren gemäß der Ansprüche 1 und 2 sowie durch entsprechende Edelmetallhuminate gemäß der Ansprüche 3 und 4. Die erfindungsgemäßen Edelmetallhuminate eignen sich insbesondere als Stabilisatoren für wäßrige Alkalihuminat-Präparate, Silber-Huminat als Breitbandbiozid, Gold-Huminat zur Therapie von rheumatischen Krankheiten und Platin-Huminat zur Behandlung maligner Tumore, entsprechend der Ansprüche 5 bis 8.

Während die Umsetzung von Edelmetallverbindungen mit Huminsäure oder Huminaten nicht zur stabilen Einbindung der Edelmetallkomponenten führt, gelingt das dauerhafte Einbringen der Edelmetallkomponente bei bestimmten Syntheseverfahren von Huminaten und zwar bei der elektrochemischen und der plasmachemischen Oxidation von alkalischen, wäßrigen Lösungen mehrwertiger phenolischer Verbindungen, wenn eine Lösung oder ein Hydrosol eines Edelmetallsalzes zu Beginn der Oxidationsreaktion der Lösung der mehrwertigen phenolischen Verbindung(en) in der Anoden- oder Reaktionskammer zugesetzt wird.

Bei diesen Syntheseverfahren bilden sich stabile Verbindungen der frisch synthetisierten Huminsäuren mit Edelmetallen, die Edelmetallhuminate. Bis zu einem Edelmetallgehalt von 5 Gew.-% sind diese Huminate in wäßriger Lösung bei Raumtemperatur mehr als ein halbes Jahr stabil. Auch bei einem Kurzzeit-Stabilitätstest, einer 24-stündigen Temperaturbelastung von 90 °C zeigen diese Lösungen keine Veränderung.

Die elektrochemische und die plasmachemische Oxidation von mehrwertigen phenolischen Verbindungen zur Herstellung niedermolekularer Alkali-Huminate ist aus EP-B 0 281 678, DE-A 41 34 379 und DE-A 41 34 384 bekannt. Die dort beschriebenen Verfahrensvarianten und Einsatzprodukte können auch zur Herstellung der erfindungsgemäßen Edelmetallhuminate eingesetzt werden. Jedoch ist die in diesen Schriften ebenfalls offenbarte Synthese durch chemische Oxidation mehrwertiger phenolischer Verbindungen nicht zur Herstellung stabiler Edelmetallhuminate geeignet.

Ausgangsprodukte für das erfindungsgemäße Verfahren können alle gängigen mehrwertigen Phenole sein, wie Brenzkatechin, Resorcin, Hydrochinon, Orcin, Gallussäure, Protocatechusäure, Pyrogallol, 2-Oxyhydrochinon, Phloroglucin oder Tetraoxybenzole.

Desgleichen können als mehrwertige phenolische Verbindungen solche der allgemeinen Formel 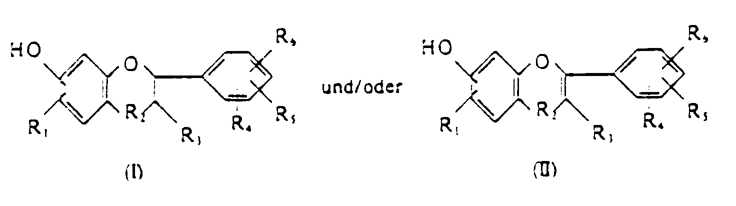
eingesetzt werden, wobei R₁, R₃ und R₄ gleich oder verschieden sind und ein OH-Gruppe oder Wasserstoff darstellen, R₂ eine CO- oder CH₂-Gruppe bedeutet und R₅ und R₆ gleich oder verschieden sind und Wasserstoff, eine OH- oder Methoxygruppe darstellen, wobei R₄, R₅ und R₆ nicht alle gleichzeitig Wasserstoff sind.

Beispiele für derartige Verbindungen sind:
Narigenin
Eriodictyol
Hesperitin
Pelargonidin
Cyanidin
Delphinidin
Päonidin
Syringidin
Catechin, bzw. Epicatechin
Gallocatechin
Apigenin
Fisetin
Robinetin
Gossypetin
Luteolin
Kämpferol
Quercetin
Morin oder Myricetin

Bevorzugte Bausteine sind Quercetin und Catechin.

Die mehrwertigen Phenole können als Reinsubstanzen oder im beliebigen Gemisch miteinander eingesetzt werden. Sie werden in Form 1 bis 5 %iger wäßriger Lösungen vorgelegt.

Als Edelmetallkomponenten können alle wasserlöslichen Salze in Form wäßriger Lösungen oder Hydrosole der Edelmetalle Gold, Silber, Quecksilber, Rhenium sowie der Platinmetalle Ruthenium, Rhodium, Palladium, Osmium, Iridium und Platin eingesetzt werden.

Die Hydrosole sind handelsübliche Produkte, die kolloidales Edelmetall mit einem Schutzkolloid (meist Gummiarabicum) enthalten.

Zur Umsetzung werden die mehrwertigen Phenole in einer alkalischen, wäßrigen Lösung gelöst. Als Laugen können alle Alkalimetallhydroxide, Ammoniak oder starke Amine in wäßriger Lösung verwendet werden. Aus wirtschaftlichen Gründen sind Natrium und Kaliumhydroxid bevorzugt.

Zur Herstellung von Huminaten mit einem mittleren Molekulargewicht von 1 000 D bei einem Streubereich von 300 bis 1 500 D wird die eingesetzte Alkalimenge gemäß der Lehre der EP-B 0 281 678 im Bereich der 1,2 bis 1,6-fachen stöchiometrischen Menge liegen, die zur Phenolatbildung der mehrwertigen Phenole, das heißt zur Neutralisation aller phenolischen OH-Gruppen notwendig ist. Dabei stellt sich ein pH-Wert der Reaktionslösung von 8 bis 9 ein.

Bei der anschließenden Oxidation tritt, auch in Anwesenheit von Edelmetallverbindungen eine automatische Begrenzung der Reaktion bei Produkten mit einem mittleren Molekulargewicht von 1 000 D bei einem Streubereich von 300 bis 1 500 D ein.

Zur Herstellung physiologisch wirkender, nicht mutagener Huminate mit einem Molekulargewicht bis etwa 50 000 D, entsprechend DE-A 41 34 379 wird die Alkalimenge so eingestellt, daß der pH-Wert des Reaktionsmediums während der Oxidation im Bereich von pH 8,5 bis 11 liegt. Die Stromzufuhr wird so dosiert, daß der Gehalt der den mehrwertigen Phenolen entsprechenden Chinonen immer kleiner als 0,5 % ist, bezogen auf die eingesetzten mehrwertigen Phenole, und daß die Oxidationsreaktion abgebrochen wird, wenn die Chinonkonzentration trotz Stromzufuhr absinkt, bzw. wenn die Chinonkonzentration trotz Stromzufuhr unter 0,05 %, bezogen auf die eingesetzten mehrwertigen Phenole, abgesunken ist.

Die elektrochemische Oxidation erfolgt in einem elektrochemischen Reaktor, wobei die Oxidationsgeschwindigkeit durch Einstellung der Anodenspannung und der Stromdichte bestimmt wird.

Die Anodenspannung kann im Bereich von 1 bis 20 V und die Stromdichte im Bereich von 0,5 bis 4 A/cm² variiert werden.

Die plasmachemische Oxidation erfolgt in einer an sich bekannten Apparatur zur Coronaentladung, wobei die Oxidationsgeschwindigkeit durch Einstellung der Betriebsspannung und der Feldstärke bestimmt wird. Die Spannung kann im Bereich von 20 bis 250 kV bei Frequenzen von 16²/₃ bis 400 Hz und die Feldstärke von 2 bis 80, bevorzugt von 10 bis 20 kV/cm variiert werden. Der Druck innerhalb der Entladungsapparatur beträgt 0,05 bis 3 kpa. Der Betrieb ist sowohl mit Sauerstoff als auch mit Luft möglich.

Sowohl die elektrochemische als auch die plasmachemische Oxidation wird im Temperaturbereich von 15 bis 55 °C, bevorzugt von 30 bis 50 °C durchgeführt. Die Temperatur des Plasmas beträgt dabei 150 bis 300 °C.

Sofort nach dem Start der Oxidationsreaktion, wenn die Reaktionsmischung beginnt braun zu werden, wird dem Reaktionsgemisch bei der elektrochemischen Oxidation in der Anodenkammer, bei der plasmachemischen Reaktion in der Reaktionsröhre eine wäßrige Lösung oder ein Hydrosol eines entsprechenden Edelmetallsalzes zugegeben, in einer Menge, daß die Konzentration des Edelmetalls im neu entstandenen Reaktionsgemisch im Bereich von 1 bis 5 Gew.-% liegt.

Nach Beendigung der Oxidationsreaktion wird der pH-Wert der Reaktionsmischung auf einen Wert im Bereich von 4 bis 6 eingestellt und ggf. gepuffert.

Dies erfolgt entweder durch Zugabe von Säure oder durch Einwirkung von saurem Ionenaustauscher und/oder nachfolgender Zugabe einer entsprechenden Pufferlösung (z. B. Phosphat-, Tris- oder Zitronensäure-Puffer).

Sofern die neutralisierte und gepufferte Lösung unerwünschte Schwebstoffe enthält, wird sie nun durch ein Trennverfahren wie Zentrifugieren (10 000 bis 30 000 xg) oder Filtrieren durch ein sehr feinporiges Filtermaterial von diesen Schwebstoffen befreit. Obwohl diese Lösung für viele Anwendungszwecke direkt eingesetzt werden kann, sollte sie bei Verwendung der Produkte als Heilmittel durch an sich bekannte Reinigungsverfahren wie präparative Chromatographiemethoden, Ultrafiltration, Ultrazentrifugation oder Elektrodialyse gereinigt und von unerwünschten Nebenprodukten befreit werden.

Erhalten wird eine Lösung, die etwa 2 bis 8 % Edelmetallhuminate enthält. Diese Lösung kann direkt verwendet werden oder durch vorsichtiges Entfernen von Wasser, etwa mittels Gefriertrocknung zu einer 20 %igen Lösung aufkonzentriert werden.

Die erhaltene und die aufkonzentrierte Lösung ist stabil.

### BEISPIELE Ziffern in [] sind CAS-Nummern

### Beispiel 1

100 g einer wäßrigen Mischung von Hydrochinon (3 % w/w) werden mit KOH auf pH 9 eingestellt. Die Mischung wird in die Anodenkammer einer elektrochemischen Zelle eingefüllt, die Katodenkammer wird mit einer wäßrigen KOH-Lösung von pH 9 beschickt. Die Zelle wird mit einer Gleichspannung von U = 2,5 Volt betrieben, der pH-Wert wird durch Zudosieren von 1 %iger KOH konstant gehalten. Beim Beginn der Oxidationsreaktion, erkennbar durch Braunfärbung der Reaktionsmischung, werden in die Anodenkammer 2 ml einer wäßrigen Lösung von Silberlaktat (0,14 g [128-00-7] eindosiert. Nach Zugabeende wird die anodische Oxidation für 12 Stunden weitergeführt, danach wird die Reaktionsmischung mit einem geeigneten sauren Ionenaustauscher neutralisiert und auf pH 5 eingestellt. Es entsteht eine klare, braune Lösung, die auch bei Temperaturbelastung von 90 °C während 24 Stunden stabil bleibt.

Der Silbergehalt beträgt 2,5 %.

### Beispiel 2

Analog zu Beispiel 1 mit Pyrogallol und Silberlaktat [128-00-7] als Edukte.

Der Silbergehalt des Huminats beträgt 2,3 %.

### Beispiel 3

Analog zu Beispiel 1 mit 2 g/100 ml Hydrochinon und 0,075 g/2 ml Tetrachlorogold-(III)-säure Trihydrat [16961-25-4] als Edukte.

Der Goldgehalt des Huminats beträgt 1,8 %.

### Beispiel 4

Analog zu Beispiel 1 mit 4 g/100 ml Pyrogallol und 0,13 g/2 ml Tetrachlorogold-(III)-säure Trihydrat [16961-25-4] als Edukte.

Der Goldgehalt des Huminats beträgt 1,6 %.

### Beispiel 5

Analog zu Beispiel 1 mit 5 g/100 ml Hydrochinon und 0,18 g/2 ml Hexachloroplatin-(IV)-säure Hydrat [16941-12-1] als Edukte.

Der Platingehalt des Huminats beträgt 1,3 %.

### Beispiel 6

Analog zu Beispiel 1 mit 3,5 g/100 ml Pyrogallol und 0,13 g/2 ml Hexachloroplatin-(IV)-säure Hydrat [16941-12-1] als Edukte.

Der Platingehalt des Huminats beträgt 1,4 %.

### Beispiel 7

In eine Niedertemperaturplasmaröhre, mit Sauerstoff bei 0,1 hPa und 15 kV/cm betrieben, werden 100 ml einer wäßrigen Mischung von Hydrochinon (3 % w/w), die mit KOH auf pH 9,5 eingestellt ist, eindosiert. Die Mischung durchläuft die Entladungszone, wird am unteren Ende der Röhre gesammelt, durch Zudosieren von 1 %iger KOH-Lösung wieder auf pH 9,5 eingestellt und dem Vorratsgefäß erneut zugeführt.

Sobald die Oxidationsreaktion, erkennbar durch Braunfärbung der Reaktionsmischung, einsetzt, werden 2 ml einer wäßrigen Lösung von Silberlaktat (0,14 g) [128-00-7] zudosiert. Nach Zugabeende wird die Reaktion für 5 Stunden weitergeführt, dann wird die Mischung mit einem geeigneten sauren Ionenaustauscher neutralisiert. Es entsteht eine klare braune Lösung, die auch bei Temperaturbelastung von 90 °C während 24 Stunden stabil bleibt.

Der Silbergehalt beträgt 2,6 %.

### Beispiel 8

Analog zu Beispiel 7 mit 3,7 9/100 ml Pyrogallol und 0,16 g/2 ml Silberlaktat [128-00-7] als Edukte.

Der Silbergehalt des Huminats beträgt 2,3 %

### Beispiel 9

Analog zu Beispiel 7 mit 3,5 g/100 ml Hydrochinon und 0,12 9/2 ml Tetrachlorogold-(III)-säure Trihydrat [1691-25-4] als Edukte.

Der Goldgehalt des Huminats beträgt 1,6 %.

### Beispiel 10

Analog zu Beispiel 7 mit 4,5 9/100 ml Pyrogallol und 0,12 g/2 ml Tetrachlorogold-(III)-säure Trihydrat [1691-25-4] als Edukte.

Der Goldgehalt des Huminats beträgt 1,3 %.

### Beispiel 11

Analog zu Beispiel 7 mit 5 9/100 ml Hydrochinon und 0,15 g/2 ml Hexachloroplatin-(IV)-säure Hydrat [16941-12-1] als Edukte.

Der Platingehalt des Huminats beträgt 1,1 %.

### Beispiel 12

Analog zu Beispiel 7 mit 2,2 9/100 ml Pyrogallol und 0,08 g/2 ml Hexachloroplatin-(IV)-säure Hydrat [16941-12-1] als Edukte.

Der Platingehalt des Huminats beträgt 1,3 %.

## Patentansprüche

1. Verfahren zur Herstellung von Edelmetallhuminaten, **dadurch gekennzeichnet**, daß eine mehrwertige phenolische Verbindung in alkalischer wäßriger Lösung elektrochemisch oxidiert wird, wobei in der Anodenkammer nach dem Start der Oxidationsreaktion der alkalischen Lösung der mehrwertigen phenolischen Verbindung eine Lösung oder ein Hydrosol eines entsprechenden Edelmetallsalzes zugegeben und das Reaktionsgemisch nach Beendigung der Oxidationsreaktion auf einen pH-Wert im Bereich von 4 bis 6 eingestellt wird.

2. Verfahren zur Herstellung von Edelmetallhuminaten, **dadurch gekennzeichnet**, daß eine mehrwertige phenolische Verbindung in alkalischer wäßriger Lösung plasmachemisch oxidiert wird, wobei in der Reaktionsröhre zu Beginn der Oxidationsreaktion der Lösung der mehrwertigen phenolischen Verbindung eine Lösung oder ein Hydrosol eines entsprechenden Edelmetallsalzes zugegeben und das Reaktionsgemisch nach Beendigung der Oxidationsreaktion auf einen pH-Wert im Bereich von 4 bis 6 eingestellt wird.

3. Edelmetallhuminate von niedermolekularen Huminsäuren mit einem Edelmetallgehalt von 1 bis 5 Gew.-%.

4. Verbindungen der Elemente Gold, Ruthenium, Rhodium, Palladium, Osmium, Iridium und Platin mit Huminsäuren mit einem mittleren Molekulargewicht von 900 bis 30 000.

5. Verwendung der Edelmetallhuminate als Stabilisatoren für wäßrige Alkali-Huminat-Präparate.

6. Verwendung von Silber-Huminat als Breitbandbiozid.

7. Verwendung von Gold-Huminat zur Herstellung von Wirkstoffen zur Therapie von rheumatischen Krankheiten.

8. Verwendung von Huminaten der Platinelemente zur Herstellung von Arzneistoffen zur Behandlung maligner Tumore.
